# EUROPEAN PATENT APPLICATION

(11) **EP 4 656 126 A1**
(43) Date of publication of application: **03.12.2025**
(21) Application number: 25178550.7
(22) Date of filing: 23.05.2025
(51) Int. Cl.: A61B 5/00, A61B 5/08, A61B 5/11, A61B 5/113

(54) **APPARATUS FOR DETECTING RESPIRATION, AND MEDICAL IMAGING DEVICE**

(30) Priority: 27.05.2024 CN 202410668499
(71) Applicant: Siemens Healthineers AG, 91301 Forchheim (DE)
(72) Inventor: BAI, Yun Tong, Shanghai, 201318 (CN); CHEN, Kai, Shanghai, 200060 (CN); WANG, Yang, Shanghai, 200127 (CN); TIAN, Yi, Shanghai, 200126 (CN)
(74) Representative: HKW Intellectual Property PartG mbB

(57) **Abstract**

The present invention discloses an apparatus for detecting respiration, and a medical imaging device. The apparatus for detecting respiration comprises: a signal acquisition unit, configured to continuously acquire RGB signals of a subject; and a signal processing unit, configured to receive the RGB signals, and determine a respiration phase, amplitude and frequency of the subject. The apparatus for detecting respiration and the medical imaging device of the present invention can automatically capture respiration signals of the subject and send same synchronously to a physician in a control room, for the purpose of various types of CT scan, without the need to introduce invasive or contact-type equipment. Many scanning protocols involve regions of the body that are affected by respiratory motion, so ensuring that the patient holds his breath in the exposure stage is crucial for image quality. Advanced applications may be provided according to the respiration signals acquired; apart from visualized respiration signals, warnings may be issued to the physician when the patient is unable to hold his breath. In addition, cost effectiveness is a major advantage, as the system is able to use an existing surveillance camera.

## Description

### TECHNICAL FIELD

The present invention relates to respiration detection.

### BACKGROUND ART

Respiration signals are an important physiological marker in CT (computed tomography) scans. In many scanning schemes involving regions of the body that are affected by respiration, failure to hold one's breath during exposure will result in serious respiratory motion artefacts, which hinder the diagnostic process. Due to radiation exposure, the operator will generally stay in the control room during the scan, so is unable to check whether the patient has successfully held his breath. If the patient's respiration status is not monitored qualitatively/quantitatively, the physician will be unable to discover problems of this kind when the scanning process has ended, and so will have no choice but to make a diagnosis with impaired image quality, or call the patient back to repeat the scan.

A conventional respiration monitoring system consists of an invasive/skin-contact sensor, but such a solution is not very practical for conventional CT scanning, for the following reasons: installing an additional device on the patient's body will not only cause the patient discomfort, but might also affect image quality, as the position where it is installed might overlap the CT scanning range; and the installation of these devices requires additional time, which will prolong the scanning time.

Apart from radiotherapy (RT) planning and treatment, there is no active respiration monitoring in ordinary CT scanning programs. In the case of CT radiotherapy planning and treatment, existing solutions for quantitative monitoring of respiration rely on a set of independent devices in contact with the patient's thoracic cavity and abdomen. The main idea is to capture fluctuations in body volume caused by respiration (rising and falling of the chest/abdominal wall). A typical example is the Varian respiratory gating system, which mainly consists of a reflector positioned on the patient's body, a camera (mounted on the PHS, wall or ceiling) and a set of auxiliary devices. When system preparations are completed, the physician must clean the reflecting plate and place it in a specific position on the patient's body, and give guidance to the patient in the course of scanning, in order to obtain a scan that is in synchrony with respiration. Other solutions are set up in a similar way, but use a hook-and-loop strap in place of the reflecting plate and camera, being fixed on the patient's abdomen to capture respiration signals. Such an apparatus has high resolution but requires additional steps (which are time-consuming), and also requires the physician to undergo special training; furthermore, the additional equipment placed on the patient's body will make the patient feel somewhat uncomfortable, so might not be suitable for all patients. Thus, such solutions are not very practical for conventional CT scans.

Chinese invention patent application CN 109862824 A has disclosed the use of an optical camera and projection shadows for the detection and measurement of respiration. US invention patent application US 2023/0248268 Al has disclosed camera-based, respiration-triggered medical scanning.

### SUMMARY OF THE INVENTION

In view of the above, the present invention proposes an apparatus for detecting respiration, and a medical imaging device.

According to a first aspect of the present invention, an apparatus for detecting respiration is provided, comprising: a signal acquisition unit, configured to continuously acquire RGB signals of a subject; and a signal processing unit, configured to receive the RGB signals, and determine a respiration phase, amplitude and frequency of the subject.

In an embodiment, the signal processing unit comprises: a chest-and-abdomen RGB signal extraction unit, configured to extract a chest-and-abdomen RGB signal from the RGB signals; a light intensity optical flow detection unit, configured to detect information on optical flow and a change in light intensity of an upper body of the subject according to the chest-and-abdomen RGB signal; and a judgment unit, configured to determine a respiration phase, amplitude and frequency of the subject according to the information on optical flow and the change in light intensity.

In an embodiment, the signal processing unit comprises: a face RGB signal extraction unit, configured to extract a face RGB signal from the RGB signals; a facial skin detection unit, configured to detect an RGB change in facial skin of the subject according to the face RGB signal; and a judgment unit, configured to determine a respiration phase, amplitude and frequency of the subject according to the RGB change in the facial skin of the subject.

In an embodiment, the signal acquisition unit is further configured to continuously acquire depth signals of the subject.

In an embodiment, the signal processing unit comprises: a chest-and-abdomen depth signal extraction unit, configured to extract a chest-and-abdomen depth signal from the depth signals; a body volume detection unit, configured to detect a change in body volume of an upper body of the subject according to the chest-and-abdomen depth signal; and a judgment unit, configured to determine a respiration phase, amplitude and frequency of the subject according to the change in body volume of the upper body of the subject.

In an embodiment, the signal processing unit comprises: a neck depth signal extraction unit, configured to extract a neck depth signal from the depth signals; a neck motion detection unit, configured to detect motion of a neck of the subject according to the neck depth signal; and a judgment unit, configured to determine a respiration phase, amplitude and frequency of the subject according to the motion of the neck of the subject.

In an embodiment, the signal acquisition unit is further configured to continuously acquire infrared signals of the subject, and the signal processing unit receives the infrared signals and uses same to assist in identifying a region of the subject.

In an embodiment, the apparatus further comprises a notification unit, configured to display respiration phase and amplitude signals of the subject and give corresponding warnings or recommendations.

According to a second aspect of the present invention, a medical imaging device is provided, comprising the apparatus described above.

In an embodiment, the medical imaging device is a CT machine, an MR machine or an X-ray machine.

The apparatus for detecting respiration and the medical imaging device of the present invention can automatically capture respiration signals of the subject and send same synchronously to a physician in a control room, for the purpose of various types of CT scan, without the need to introduce invasive or contact-type equipment. Many scanning protocols involve regions of the body that are affected by respiratory motion, so ensuring that the patient holds his breath in the exposure stage is crucial for image quality. Advanced applications may be provided according to the respiration signals acquired; apart from visualized respiration signals, warnings may be issued to the physician when the patient is unable to hold his breath. In addition, cost effectiveness is a major advantage, as the system is able to use an existing surveillance camera.

### BRIEF DESCRIPTION OF THE DRAWINGS

Preferred embodiments of the present invention will be described in detail below with reference to the drawings, to give an ordinary person skilled in the art a clearer understanding of the abovementioned and other features and advantages of the present invention. In the drawings:
Fig. 1 is a schematic structural block diagram of an apparatus for detecting respiration according to an embodiment of the present invention.
Fig. 2 is a schematic picture of an image acquired by the signal acquisition unit of the apparatus for detecting respiration shown in Fig. 1.
Fig. 3 is a schematic structural block diagram of the signal processing unit of the apparatus for detecting respiration shown in Fig. 1.

The reference labels used in the abovementioned drawings are as follows:

| | | | |
|---|---|---|---|
| 100 | Apparatus for detecting respiration | 124 | Neck region |
| 102 | Gantry | 126 | Chest-and-abdomen region |
| 104 | Bed board | 128 | Chest-and-abdomen RGB signal extraction unit |
| 106 | Subject | 130 | Light intensity optical flow detection unit |
| 108 | Signal acquisition unit | 132 | Judgment Unit |
| 110 | Ceiling | 134 | Face RGB signal extraction unit |
| 112 | Upright post | 136 | Facial skin detection unit |
| 114 | Wall | 138 | Chest-and-abdomen depth signal extraction unit |
| 116 | Signal processing unit | 140 | Body volume detection unit |
| 118 | Notification unit | 142 | Neck depth signal extraction unit |
| 120 | Image | 144 | Neck motion detection unit |
| 122 | Face region | | |

### DETAILED DESCRIPTION OF EMBODIMENTS

To clarify the objectives, technical solutions and advantages of the present invention, the present invention will be explained in further detail below through embodiments.

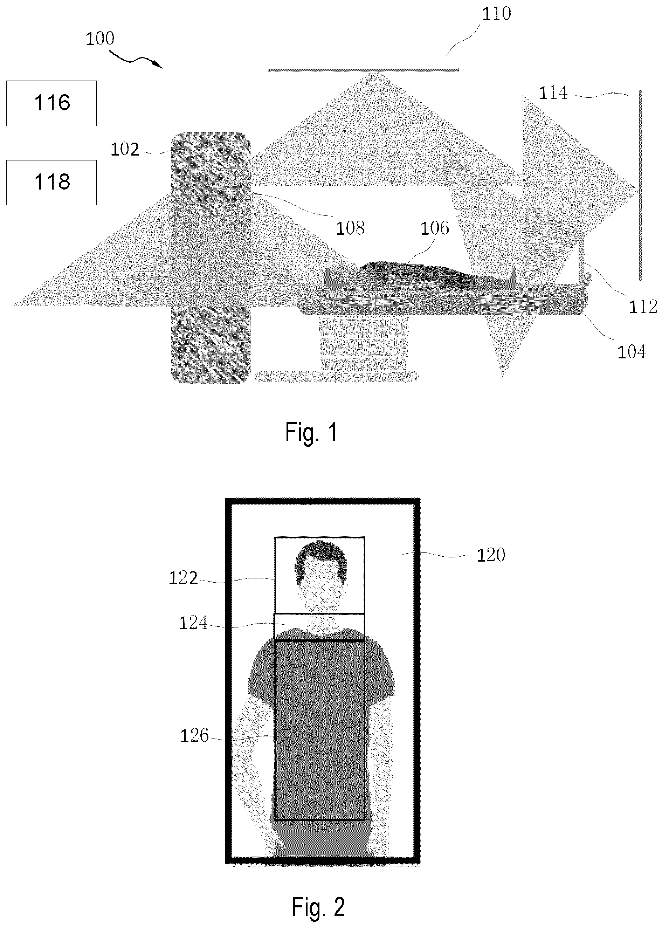

Fig. 1 is a schematic structural block diagram of an apparatus 100 for detecting respiration according to an embodiment of the present invention. As shown in Fig. 1, the apparatus 100 for detecting respiration comprises a signal acquisition unit 108 and a signal processing unit 116. The apparatus 100 for detecting respiration may be part of a medical imaging device. In this embodiment, the medical imaging device is a CT machine. In other embodiments, the medical imaging device may be an MR (magnetic resonance) machine or an X-ray machine, etc. The medical imaging device comprises a gantry 102 and a bed board 104, the bed board 104 being used to carry a subject 106, for example a human body. The signal acquisition unit 108 may for example be a 2D or 3D camera, configured to continuously acquire RGB signals of the subject 106. The signal acquisition unit 108 may be arranged in front of or behind the gantry 102, or arranged on a ceiling 110, or fixed to an upright post 112 of the bed board 104, or to a wall 114.

The signal processing unit 116 is configured to receive the RGB signals, and determine a respiration phase, amplitude and frequency of the subject 106.

The signal acquisition unit 108 continuously acquires an RGB stream. The exact position and angle at which the signal acquisition unit 108 is mounted might vary, depending on the type of the signal acquisition unit 108 and the sizes of the gantry 102, examination table and scanning room. Different mounting choices will result in different fields of view. Apart from mounting on the bed board 104, other positions will change the relative positions of the signal acquisition unit 108 and the patient during scanning, and consequently it might be necessary to perform corresponding standardization. Typical forms of standardization include distortion correction, image size adjustment, etc. Fig. 2 is a schematic picture of an image 120 acquired by the signal acquisition unit 108 of the apparatus 100 for detecting respiration shown in Fig. 1. The image 120 has been standardized, having good coverage of the subject's face, neck and upper body. As shown in Fig. 2, the image 120 is identified as having a face region 122, a neck region 124 and a chest-and-abdomen region 126. Due to differences in the way the signal acquisition unit 108 is mounted, the signal acquisition unit 108 might be unable to completely cover the head, neck and upper body during scanning.

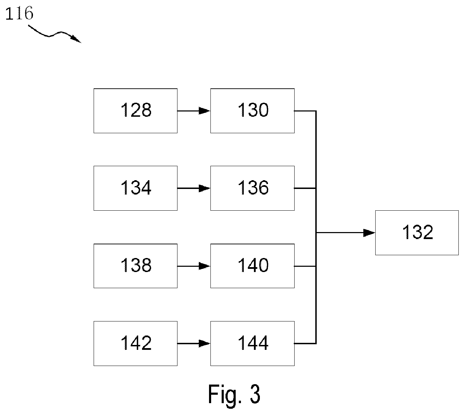

Fig. 3 is a schematic structural block diagram of the signal processing unit 116 of the apparatus 100 for detecting respiration shown in Fig. 1.

As shown in Fig. 3, the signal processing unit 116 may comprise a chest-and-abdomen RGB signal extraction unit 128, a light intensity optical flow detection unit 130 and a judgment unit 132. The chest-and-abdomen RGB signal extraction unit 128 is configured to extract a chest-and-abdomen RGB signal from the RGB signals, for example from the chest-and-abdomen region 126 in Fig. 2. The light intensity optical flow detection unit 130 is configured to detect a change in light intensity of the upper body of the subject 106 according to the chest-and-abdomen RGB signal. Light intensity can measure a change in body volume of the upper body, to reflect inhalation and exhalation. The judgment unit 132 is configured to determine a respiration phase, amplitude and frequency of the subject 106 according to the change in light intensity.

As shown in Fig. 3, the signal processing unit 116 may also comprise a face RGB signal extraction unit 134, a facial skin detection unit 136 and the judgment unit 132. The face RGB signal extraction unit 134 is configured to extract a face RGB signal from the RGB signals, for example from the face region 122 in Fig. 2. The face RGB signal comprises RGB changes in the facial skin caused by blood vessel motion, heartbeat and oxygen-rich blood circulation. The facial skin detection unit 136 is configured to detect an RGB change in the facial skin of the subject 106 according to the face RGB signal. The judgment unit 132 is configured to determine a respiration phase, amplitude and frequency of the subject 106 according to the RGB change in the facial skin of the subject 106.

In this embodiment, the signal acquisition unit 108 is a 3D camera, and the signal acquisition unit 108 is further configured to continuously acquire depth signals of the subject 106. As shown in Fig. 3, the signal processing unit 116 may also comprise a chest-and-abdomen depth signal extraction unit 138, a body volume detection unit 140 and the judgment unit 132. The chest-and-abdomen depth signal extraction unit 138 is configured to extract a chest-and-abdomen depth signal from the depth signals. The chest-and-abdomen depth signal can measure a change in body volume of the upper body by means of a dimension difference, to reflect inhalation and exhalation. The body volume detection unit 140 is configured to detect a change in body volume of the upper body of the subject 106 according to the chest-and-abdomen depth signal. The judgment unit 132 is configured to determine a respiration phase, amplitude and frequency of the subject 106 according to the change in body volume of the upper body of the subject 106.

As shown in Fig. 3, the signal processing unit 116 may also comprise a neck depth signal extraction unit 142, a neck motion detection unit 144 and the judgment unit 132. The neck depth signal extraction unit 142 is configured to extract a neck depth signal from the depth signals. The depth signal can capture motion of the neck or trachea caused by respiration. The neck motion detection unit 144 is configured to detect motion of the neck of the subject 106 according to the neck depth signal. The judgment unit 132 is configured to determine a respiration phase, amplitude and frequency of the subject 106 according to the motion of the neck of the subject 106.

In this embodiment, the signal acquisition unit 108 is further configured to continuously acquire infrared signals of the subject 106, and the signal processing unit 116 receives the infrared signals and uses same to assist in identifying a region of the subject 106.

The face RGB signal, chest-and-abdomen RGB signal, chest-and-abdomen depth signal and neck depth signal may be used to extract respiration information independently, but may also supplement and confirm each other. Depending on the availability and completeness of these data, joint estimation of respiration phase, amplitude and frequency can be performed. The extracted respiration phase, amplitude and frequency may be used to optimize a workflow of the medical imaging device.

There are certain factors that might influence the usability of the RGB, depth and infrared signals. For example, if the subject has a face covering such as a mask or spectacles, facial recognition performed with RGB signals might fail, even with the assistance of infrared signals. If the subject is wearing loose clothing, or has a sheet covering his body, it will be nearly impossible to recover the required depth signal of the chest and abdomen.

As shown in Fig. 1, the apparatus 100 for detecting respiration may further comprise a notification unit 118, configured to display respiration phase and amplitude signals of the subject 106 and give corresponding warnings or recommendations. For example, respiration signals may be visualized, and corresponding criteria may be used to generate alerts for the physician according to different scanning stages, to prevent respiration from affecting image quality.

The apparatus for detecting respiration and the medical imaging device of the present invention can automatically capture respiration signals of the subject and send same synchronously to a physician in a control room, for the purpose of various types of CT scan, without the need to introduce invasive or contact-type equipment. Many scanning protocols involve regions of the body that are affected by respiratory motion, so ensuring that the patient holds his breath in the exposure stage is crucial for image quality. Advanced applications may be provided according to the respiration signals acquired; apart from visualized respiration signals, warnings may be issued to the physician when the patient is unable to hold his breath. In addition, cost effectiveness is a major advantage, as the system is able to use an existing surveillance camera.

The above are merely preferred embodiments of the present invention, which are not intended to limit it. Any amendments, equivalent substitutions or improvements etc. made within the spirit and principles of the present invention shall be included in the scope of protection thereof.

## Claims

1. An apparatus (100) for detecting respiration, comprising:
a signal acquisition unit (108), configured to continuously acquire RGB signals of a subject (106);
a signal processing unit (116), configured to receive the RGB signals, and determine a respiration phase, amplitude and frequency of the subject (106).

2. The apparatus (100) as claimed in claim 1, wherein the signal processing unit (116) comprises:
a chest-and-abdomen RGB signal extraction unit (128), configured to extract a chest-and-abdomen RGB signal from the RGB signals;
a light intensity optical flow detection unit (130), configured to detect information on optical flow and a change in light intensity of an upper body of the subject (106) according to the chest-and-abdomen RGB signal;
a judgment unit (132), configured to determine a respiration phase, amplitude and frequency of the subject (106) according to the information on optical flow and the change in light intensity.

3. The apparatus (100) as claimed in claim 1, wherein the signal processing unit (116) comprises:
a face RGB signal extraction unit (134), configured to extract a face RGB signal from the RGB signals;
a facial skin detection unit (136), configured to detect an RGB change in facial skin of the subject (106) according to the face RGB signal;
a judgment unit (132), configured to determine a respiration phase, amplitude and frequency of the subject (106) according to the RGB change in the facial skin of the subject (106).

4. The apparatus (100) as claimed in claim 1, wherein the signal acquisition unit (108) is further configured to continuously acquire depth signals of the subject (106).

5. The apparatus (100) as claimed in claim 4, wherein the signal processing unit (116) comprises:
a chest-and-abdomen depth signal extraction unit (138), configured to extract a chest-and-abdomen depth signal from the depth signals;
a body volume detection unit (140), configured to detect a change in body volume of an upper body of the subject (106) according to the chest-and-abdomen depth signal;
a judgment unit (132), configured to determine a respiration phase, amplitude and frequency of the subject (106) according to the change in body volume of the upper body of the subject (106).

6. The apparatus (100) as claimed in claim 4, wherein the signal processing unit (116) comprises:
a neck depth signal extraction unit (142), configured to extract a neck depth signal from the depth signals;
a neck motion detection unit (144), configured to detect motion of a neck of the subject (106) according to the neck depth signal;
a judgment unit (132), configured to determine a respiration phase, amplitude and frequency of the subject (106) according to the motion of the neck of the subject (106).

7. The apparatus (100) as claimed in claim 1, wherein the signal acquisition unit (108) is further configured to continuously acquire infrared signals of the subject (106), and the signal processing unit (116) receives the infrared signals and uses same to assist in identifying a region of the subject (106).

8. The apparatus (100) as claimed in claim 1, wherein it further comprises a notification unit (118), configured to display respiration phase and amplitude signals of the subject (106) and give corresponding warnings or recommendations.

9. A medical imaging device, comprising the apparatus (100) as claimed in any one of claims 1 - 8.

10. The medical imaging device as claimed in claim 9, wherein the medical imaging device is a CT machine, an MR machine or an X-ray machine.
